(19) Europäisches Patentamt
European Patent Office
Office européen des brevets

(11) **EP 0 857 054 B1**

(12) **EUROPEAN PATENT SPECIFICATION**

(45) Date of publication and mention
of the grant of the patent:
**07.04.2004 Bulletin 2004/15**

(51) Int Cl.[7]: **A61K 7/11**, C08F 2/10,
C08F 220/60, C08F 226/10

(21) Application number: **95943039.8**

(22) Date of filing: **11.12.1995**

(86) International application number:
**PCT/US1995/015973**

(87) International publication number:
**WO 1996/019967 (04.07.1996 Gazette 1996/30)**

(54) **PROCESS FOR MAKING SUBSTANTIALLY HOMOGENEOUS COPOLYMERS OF VINYL PYRROLIDONE AND N-3,3-DIMETHYLAMINOPROPYL METHACRYLAMIDE FOR PERSONAL CARE APPLICATIONS**

VERFAHREN ZUR ERZEUGUNG VON IM WESENTLICHEN HOMOGENEN COPOLYMEREN VON VINYLPYRROLIDON UND N-3,3-DIMETHYLAMINOPROPYLMETACRYLAMID FÜR KÖRPERPFLEGEANWENDUNGEN

PROCEDE DE FABRICATION DE COPOLYMERES PRATIQUEMENT HOMOGENES DE PYRROLIDONE DE VINYLE ET DE METHACRYLAMIDE DE N-3,3-DIMETHYLAMINOPROPYLE DESTINES A DES PRODUITS DE SOINS

(84) Designated Contracting States:
**CH DE ES FR GB LI**

(30) Priority: **28.12.1994 US 365258**
**28.12.1994 US 365384**

(43) Date of publication of application:
**12.08.1998 Bulletin 1998/33**

(73) Proprietor: **ISP INVESTMENTS INC.**
**Wilmington, Delaware 19801 (US)**

(72) Inventors:
• **LIU, Kou-Chang**
**Wayne, NJ 07470 (US)**

• **ANDERSON, Lowell, R.**
**Morristown, NJ 07960 (US)**
• **HARDY, Eugene, E.**
**East Brunswick, NJ 08816 (US)**
• **REUVEN, Yakir**
**West Orange, NJ 07052 (US)**

(74) Representative: **Harrison, David Christopher et al**
**MEWBURN ELLIS**
**York House**
**23 Kingsway**
**London WC2B 6HP (GB)**

(56) References cited:
**US-A- 2 100 900**          **US-A- 4 039 734**
**US-A- 4 923 694**

**Description**

This invention relates to a process for making substantially homogeneous copolymers of vinyl pyrrolidone (VP) and N-[3-(dimethylamino)propyl] methacrylamide (DMAPMA) of predetermined composition and clear, low viscosity aqueous solutions thereof, for use in hair styling and hair care applications.

Several synthetic polymers containing vinyl lactams are presently being used in cosmetic formulations, particularly in hair care products, to contribute body, set retention and conditioning to such products.

Representative of the art in this field are the following U.S. patents: 3,914,403; 3,954,960; 4,057,533; 4,210,161; 4,586,518; 4,753,793; 4,764,363; 4,834,968; 4,842,850; 4,902,499; 4,906,459; 4,923,694; 4,963,348; 4,983,377; 5,011,895 and 5,015,708; and WO 91/15186; WO 91/15185; EPO 0412704A2; EPO 0412707A1; and JP 57126409.

Generally these synthetic polymers were made by a "one-pot" polymerization process in which selected amounts of the several monomers were reacted together. The composition of these one-pot polymers was considered as being the same as the composition of the charged monomers. However, in reality, such a polymerization process can provide only a mixture of polymers of various compositions, and, additionally, an indeterminate amount of homopolymers and undesired copolymers. Particularly relevant analyst there is US-A-4 923 694 since it uses the same starting monomer.

Accordingly, it is an object of this invention to provide a process for making substantially homogeneous copolymers of vinyl pyrrolidone (VP) and N-[3-(dimethylamino)propyl] methacrylamide (DMAPMA).

Another object of this invention is to provide a clear, low viscosity aqueous solution of homogeneous copolymers of VP and DMAPMA, in a compositional range of 20-99% VP and 1-80% DMAPMA, preferably 50-95% VP and 5-50% DMAPMA, and most preferably about 80% VP and 20% DMAPMA, at a solids level of 5-20%, preferably about 10-15%, by weight of the solution, having a Brookfield viscosity of 5 to 80 $Nm^{-2}s$ (5,000 to 80,000 cps), preferably 10 to 70 $Nm^{-2}s$ (10,000 to 70,000 cps).

Still another object herein is to provide clear, low viscosity aqueous solutions of substantially homogeneous copolymers of VP and DMAPMA in a predetermined compositional range and at a selected solids level, which, upon formulation into hair styling compositions, will provide enhanced conditioning, fixative and hair holding properties for the user.

A process is described herein for making substantially homogeneous copolymers of 20-99% vinyl pyrrolidone and 1-80% N-[3-(dimethylamino)propyl] methacrylamide, preferably about 50-95% VP and 5-50% DMAPMA, and most preferably about 80% VP and 20% DMAPMA, by weight, having a weight average molecular weight of 200,000 to 1,500,000, and a clear, low viscosity aqueous solution of the homogeneous copolymer at a 5-20% solids level, preferably 10-15%, by weight of the solution, and having a viscosity of 5 to 80 $Nm^{-2}s$ (5,000 to 80,000 cps), preferably 10 to 70 $Nm^{-2}s^{-1}$ (10,000 to 70,000 cps).

The copolymer solution obtained herein is less viscous and has better clarity than solutions of non-homogeneous copolymers of the same composition, or of related commercial products. When formulated into hair styling compositions, these homogeneous polymer solutions also provide improved curl retention (hold) during use.

In the drawings:

FIGURE 1 is a graphical representation of a non-homogeneous (one-pot) process in which is plotted percent unreacted VP and DMAPMA vs. time for a reaction mixture of 80:20 % by weight VP/DMAPMA copolymer at 15% solids.

FIGURE 2 is a graphical representation of the homogeneous process of the invention in which is plotted percent unreacted VP and DMAPMA vs. time during the preparation of an aqueous solution of 80:20 % by weight VP/DMAPMA copolymer at 15% solids.

In accordance with the present invention, a process is provided for making substantially homogeneous copolymers of vinyl pyrrolidone and N-[3-(dimethylamino)-propyl] methacrylamide of predetermined composition.

In this process, the less reactive monomer of the copolymer (VP) is precharged into a reactor at a reaction temperature of 50-80°C., preferably 55-75°C. Then the more reactive monomer (DMAPMA) is introduced incrementally into the VP-charged reactor at a rate which corresponds to the observed rate of disappearance of VP.

The entire predetermined amount of the DMAPMA monomer is added before substantially all the VP monomer has been consumed so that both monomers can react to form a substantially homogeneous copolymer in a desired compositional ratio of VP:DMAPMA. Consequently, a copolymer is obtained whose composition approaches the nominal monomer ratio of the desired copolymer composition and whose structure has the two individual monomeric units of the copolymer distributed substantially uniformly in a homogeneous chain along the backbone of the polymer. For example, for an 80:20 weight ratio of VP/DMAPMA copolymer, which is approximately equivalent to a 6:1 mole ratio of VP/DMAPMA, the homogeneous copolymer of the invention has a monomer distribution corresponding substantially close to VP-VP-VP-VP-VP-VP-DMAPMA-VP-VP-VP-VP-VP-VP-DMAPMA ....

The precharge in the process of the invention may include some DMAPMA therein, generally in an amount which is less than 10% of the total amount of DMAPMA required for the predetermined copolymer composition without affecting the homogeneous polymerization process. However, it is still necessary that the rate of addition of DMAPMA after any precharge is carried out at substantially the rate of disappearance of VP during copolymerization.

The schedule of addition of DMAPMA to accomplish the desired matched rate of reaction of VP is determined in the following manner.

DETERMINATION OF ADDITION SCHEDULE FOR DMAPMA TO FORM A HOMOGENEOUS COPOLYMER OF VP AND DMAPMA

A. First, a one-pot (comparative) copolymerization of VP and DMAPMA was carried out as follows:

(COMPARATIVE) EXAMPLE 1

VP (215 g), DMAPMA (54 g), and deionized water (1530 g) were charged into a 2-liter resin pot equipped with a gas inlet, a liquid inlet, a thermometer and a condenser. A stream of nitrogen then was bubbled through the solution and maintained during the reaction. The solution was gradually heated to 68°C.; then 0.25 ml of Lupersol 11 (t-butylperoxy pivalate) as catalyst was added; then another 0.25 ml of the catalyst was added after 10 minutes; and another 6 units of 0.25 ml was added each 30 minutes. The total addition was carried out over a 4-hour period.

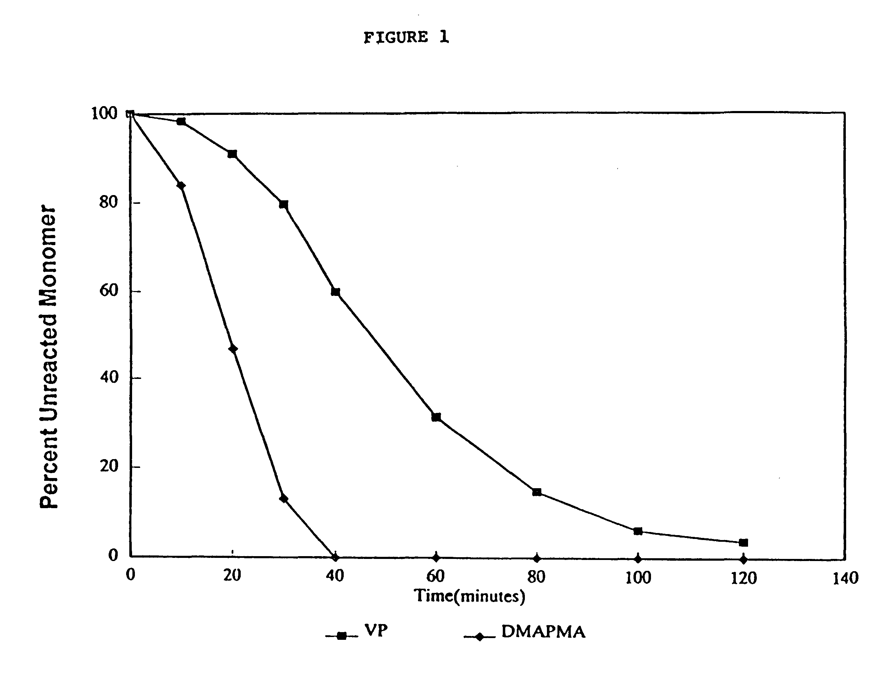

The relative percentage amounts of residual monomers present during the course of the one-pot reaction was determined by gas chromatographic analysis after sampling the reaction mixture periodically. The analytical data obtained then was plotted as the graph of FIGURE 1

As shown in FIGURE 1, DMAPMA reacts much more rapidly than VP. Accordingly, after 240 minutes, all the DMAPMA is consumed while residual VP monomer still is available for homopolymerization. Thus the copolymer formed is of a composition different from the desired monomer ratio selected by the precharged amounts of the two monomers. Under these experimental conditions, the polymer product obtained is a complex mixture of a homopolymer which is polyvinylpyrrolidone, and a copolymer of VP and DMAPMA of uncertain composition.

B. To form a homogeneous copolymer, it is necessary that the curve of rate of reaction vs. time for DMAPMA substantially coincide or match the rate of reaction curve for VP. To accomplish this, the VP is precharged and substantially all the DMAPMA is fed after to the precharge at a feeding schedule determined by analysis of the data of FIGURE 1. The % DMAPMA monomer to be fed at time t of the polymerization is determined from the Asymmetric Double Sigmoidal Distribution formula, $A_t$, below, which has four adjustable parameters, $a_1$, $a_2$, $a_3$ and $a_4$ :

$$A_t = \frac{1}{1 + \exp\left[\dfrac{a_1 - \dfrac{a_2}{2} - t}{a_3}\right]} \left[1 - \frac{1}{1 + \exp\left[\dfrac{a_1 + \dfrac{a_2}{2} - t}{a_4}\right]}\right]$$

where

t = time in minutes during copolymerization;

$a_1$ is a parameter which determines the center of the distribution;

$a_2$ is a parameter which affects the width of the distribution;

$a_3$ is a parameter which determines the ascending portion of the distribution; and

$a_4$ is a parameter which determines the descending portion of the distribution.

$$\% \text{ DMAPMA to be fed at time } t = \frac{A_t}{\sum_{t=0}^{N} A_t} \times 100$$

where N = time when the polymerization is completed.

To match the DMAPMA curve to the VP curve of FIGURE 1, an "initial guess" is made for the values of $a_1$, $a_2$, $a_3$ and $a_4$. These values are inserted into the $A_t$ formula and the % DMAPMA to be fed at time t is calculated. Then a polymerization reaction is carried out using this schedule. The resulting % unreacted DMAPMA during this polymerization will probably not match the % unreacted VP at the same time t. If the % unreacted DMAPMA at time t is too large, then the value of $a_3$ (ascendency) in the $A_t$ formula is increased, $a_4$ (descendency) is decreased, $a_1$ (center) is decreased, and $a_2$ (width) is decreased. Conversely, if the initial guess values of $a_1$ through $a_4$ give a reaction rate for DMAPMA which is too fast, then changes in the values of $a_1$ through $a_4$ are made in a direction opposite to those discussed above.

These new values of the parameters are then used to determine a new feeding schedule. Using this feeding schedule, another polymerization is carried out, and the process of adjustment of the parameters described above is repeated.

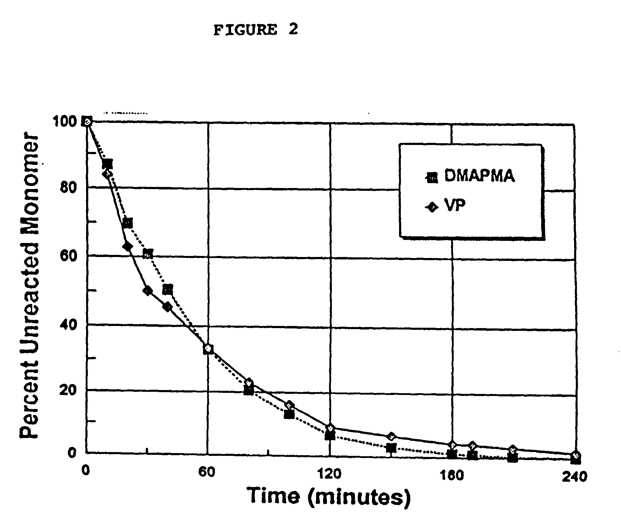

This process is known as "iterative fitting" of data to a curve. After 4 or 5 such iterative fittings, the experimental VP and DMAPMA curves will be matched to a satisfactory degree, as shown in FIGURE 2 herein.

The matching curve of DMAPMA in FIGURE 2 can originate from at least one set of values for $a_1$, $a_2$, $a_3$ and $a_4$ (the last set of the iterative fitting process) used to calculate a suitable feeding schedule of DMAPMA over the entire period of the polymerization. One such set is:

$a_1 = 17$

$a_2 = 28$

$a_3 = 2$

$a_4 = 40$

C. With such a suitable DMAPMA feeding schedule available, a homogeneous copolymer of VP and DMAPMA was prepared as described in Example 2 below.

EXAMPLE 2

Preparation of a Homogeneous Copolymer of VP and DMAPMA

In this process, 216 grams of vinylpyrrolidone (recrystallized material) and 580 grams of deionized water were charged to a 2-liter water jacketed resin flask.

The flask was equipped with an overhead stirrer connected to an anchor stirrer, a nitrogen inlet for sparging to remove dissolved air and a thermocouple which is connected to a temperature controller. The temperature was raised to 68°C., by passing heated water from a temperature controlled bath into the jacket of the resin flask. A mechanical syringe pump which could be set to deliver DMAPMA at a set rate was connected to the reactor through polyethylene tubing.

The DMAPMA reactant was added from the syringe pump as a 1 liter solution of 54 grams of DMAPMA in water, according to the following feeding schedule:

| Time (min.) | Amount of DMAPMA Solution Added (ml) |
|---|---|
| 0-30 | 418.58 |
| 30-60 | 269.80 |

(continued)

| Time (min.) | Amount of DMAPMA Solution Added (ml) |
|---|---|
| 60-90 | 153.90 |
| 90-120 | 80.56 |
| 120-150 | 40.10 |
| 150-180 | 19.38 |
| 180-210 | 9.32 |
| 210-240 | 4.38 |

The totals added during the entire reaction were VP (216 grams), DMAPMA (54 grams) and water (1530 grams).

Additions of Lupersol 11 (t-butyl peroxypivalate, Atochem, NA) initiator were made at various intervals throughout the initial part of the reaction. Additions of 0.25 ml of the initiator were made at 0, 10, 40, 70, 100, 140 and 180 minutes. At 210 and 240 minutes, 0.25 ml of either VAZO 501 (4,4'-azobis(4-cyanovaleric) acid, Wako Pure Chemical Industries, Ltd, Japan) was added to complete the reaction of any residual vinylpyrrolidone monomer. For this purpose, the reaction was allowed to proceed for a total of 10 hours. During this final period the temperature was raised to 75°C. where it was maintained until the reaction was completed.

The product was discharged as a clear, moderately viscous, aqueous solution of a substantially homogeneous copolymer of VP and DMAPMA in an 80:20 weight ratio at a 15% solids level. The residual vinylpyrrolidone content was <0.1%.

The procedure described above can be repeated to provide optimized DMAPMA feeding schedules for any selected copolymer composition, and at any particular solids content, at a prescribed polymerization temperature, solvent level, and amount of initiator.

Table 1 below shows the advantageous physical properties of low viscosity and enhanced clarity for the homogeneous copolymer solution of the invention, as compared to non-homogeneous copolymer solutions of the same composition.

TABLE 1

| | Viscosity*** $Nm^{-2}s^{-1}$ (cps) | Clarity[+] (NTU) | Mol. Wt. |
|---|---|---|---|
| Homogeneous copolymer solution | 61.5 (61,500) | 1.80* | 610,000 |
| Non-homogeneous copolymer solution | 150 (150,000) | 14.15** | 620,000 |

[+] Hach; on 0.4% aqueous solution

* pH = 7.98

** pH = 7.44

*** Brookfield viscosity, on 15% aqueous solution; Model RTV, 20 rpm, Spindle #7, 25°C.

## HAIR STYLING COMPOSITIONS

The substantially homogeneous copolymer of the invention finds particular utility in multifunctional hair care products such as water-based, rinse-off hair styling and conditioning products, and in mousse hair care products. It may be included in such compositions as a concentrate, or as a gel, and applied as a self-actuated pump hair spray, or in an aerosol product with a propellant. Various actuator and packaging devices known in the art may be used therewith.

Such hair styling products have significantly improved hair holding (curl retention) and hair stiffness properties, while maintaining enhanced hair conditioning (detangling, flyaway and combability) properties, compared to non-homogeneously prepared copolymers of the same monomers, when formulated into hair styling compositions.

| HAIR STYLING COMPOSITIONS OF INVENTION | | |
|---|---|---|
| | Hair Styling Gel | Wt (g) |
| (a) | Homogeneous copolymer of VP/DMAPMA (80:20) (as 15% aqueous solution) | 5.00 |
| (b) | Hydroxyethyl cellulose (Natrosol® 250 HHR-Aqulon) | 0.50 |
| (c) | Triethanolamine (99%) (TEA) | 0.20 |
| (d) | Deionized water | qs |

Preparation:

1. Heat (d) to 85°C.

2. Add (b) while mixing well.

3. Add (a) with rapid mixing.

4. Neutralize with (c).

| | Hair Styling Mousse | Wt (g) |
|---|---|---|
| (a) | Homogeneous copolymer of VP/DMAPMA (80:20) (as 15% aqueous solution) | 5.0 |
| (b) | Sodium cocoyl isothionate (Igepon® AC-78 Rhone-Poulenc) | 0.75 |
| (c) | Emulsifying wax (Polowax® A-31 Croda) | 0.15 |
| (d) | Propylene glycol, diazolidinyl urea, methyl paraben and propyl paraben (Germaben® II - Sutton (ISP) | 0.25 |
| (e) | Dymel® 152A (1,1-difluoroethane) | 4.00 |
| (f) | Hydrocarbon A-17 (n-butane) | 4.00 |
| (g) | Water | qs |

Preparation:

1. Add (a) to (g) while agitating and heating to 55-60°C.
2. Add (b) while mixing at 55°C. Begin cooling.
3. Add (c) and mix rapidly.
4. Cool to 25°C. and add (d).
5. Charge into pressurized container and add (e) and (f).

Table 2 below demonstrates the advantageous curl retention property for a hair styling composition (gel) containing the homogeneous copolymer of the invention as compared to a non-homogeneous copolymer.

TABLE 2

| CURL RETENTION PROPERTY | |
|---|---|
| Copolymer in Hair Styling Composition | Relative Curl Retention of Composition* |
| Homogeneous Copolymer | 116.9 |
| Non-homogeneous Copolymer | 95.3 |

* vs. standard at 90% RH and 90 min. duration

While the invention has been described with particular reference to certain embodiments thereof, it will be understood that changes and modifications may be made which are within the skill of the art.

**Claims**

1. A process for making clear aqueous solutions of a homogeneous copolymer of vinyl pyrrolidone (VP) and N-[3-(dimethylamino)propyl] methacrylamide (DMAPMA), in the compositional range of 20-99% VP and 1-80% DMAPMA, by weight, having a weight average molecular weight of 200,000 to 1,500,000, whose compositional ratio approaches the nominal monomer ratio of VP:DMAPMA and whose structure has the respective monomer units distributed along the backbone of the polymer, the solution having a solids content of 5-20%, and a Brookfield viscosity of 5 to $80 Nm^{-2}s$ (5,000 to 80,000 cps) by polymerisation of the monomers in water in the presence of a radical initiator, which comprises:

(a) precharging a reactor with VP, and water, at a polymerisation temperature of 50-80°C, and
(b) introducing DMAPMA into said reactor at a rate corresponding to the rate of disappearance of VP.

2. A process according to claim 1 wherein the DMAPMA is introduced in water.

3. A process according to claim 2 wherein said compositional range of VP:DMAPMA is 50-59% to 5-50%.

4. A process according to claim 1 wherein said solids content is 10-15%.

5. A process according to claim 4 wherein said viscosity is 10 to 70 $Nm^{-2}s$ (10,000 to 70,000 cps).

6. A process according to claim 1 which also includes the step of post-heating with additional radical initiator to reduce the residual VP content to <0.1%.

7. A process according to claim 1 wherein DMAPMA is introduced until substantially all the starting VP has been copolymerised to the desired copolymer.

8. A process according to claim 1 wherein the radical initiator is t-butylperoxy pivalate.

9. A process according to claim 6 wherein the additional radical initiator is 4,4'-azobis(4-cyano-valeric acid).

10. A process according to claim 1 wherein up to 10% of the DMAPMA is included in the precharge.

11. A clear, aqueous solution of a homogeneous copolymer of VP and DMAPMA, in the compositional range of 2-99% by weight VP and 1-80% by weight DMAPMA, having a weight average molecular weight of 200,000 to 1,500,000, whose compositional ratio approaches the nominal monomer ratio of VP:DMAPMA and whose structure has the respective monomer units distributed along the backbone of the polymer, the solution having a solids content of 5-20% and a viscosity of 10-80 $Nm^{-2}s$ (10,000 to 80,000 cps).

12. A clear, aqueous solution, according to claim 11 wherein said compositional range is 50-95% VP to 5-50% DMAPMA.

13. A clear, aqueous solution according to claim 11 wherein said solids content is 10-15%.

14. A clear, aqueous solution according to claim 13 wherein said viscosity is 10 to 70 $Nm^{-2}s$ (10,000 to 70,000 cps).

15. A clear, aqueous solution according to claim 11 wherein said compositional range is 80:20, in % by weight.

16. A hair styling composition containing an aqueous solution of a homogeneous copolymer of VP and DMAPMA in the compositional range of 20-90% VP and 1-80% DMAPMA, by weight, according to claim 12.

17. A hair styling composition according to claim 16 wherein said homogeneous copolymer is prepared according to the process of claim 1.


**Revendications**

1. Procédé pour rendre claires des solutions aqueuses d'un copolymère homogène de pyrrolidone de vinyle (PV) et de méthacrylamide de N-[3-(diméthylamino)propyle] (MADMAP), dans une gamme de composition de 20-99% de PV et 1-80% de MADMAP, en poids, ayant un poids moyen de poids moléculaire de 200000 à 1500000, dont le rapport de composition approche le rapport nominal du monomère de PV:MADMAP et dont la structure a les unités respectives du monomère distribuées le long du squelette du polymère, la solution ayant une teneur en matière sèche de 5-20%, et une viscosité de Brookfield de 5 à 80 $Nm^{-2}s$ (5000 à 80000 cps) par polymérisation des monomères dans l'eau en présence d'un radical initiateur, qui comprend:

(a) le préchargement d'un réacteur avec le PV, et l'eau, à une température de polymérisation de 50-80°C, et

(b) l'introduction de MADMAP dans ledit réacteur à un taux correspondant au taux de disparition du PV.

2. Procédé selon la revendication 1 dans lequel le MADMAP est introduit dans l'eau.

3. Procédé selon la revendication 2 dans lequel ladite gamme de composition de PV:MADMAP est de 50-59% à 5-50%.

4. Procédé selon la revendication 1 dans lequel ladite teneur en matière sèche est de 10-15%.

5. Procédé selon la revendication 4 dans lequel ladite viscosité est de 10 à 70 Nm$^{-2}$s (10000 à 70000 cps).

6. Procédé selon la revendication 1 qui inclut également une étape de postchauffage avec un radical initiateur supplémentaire pour réduire le contenu résiduel en PV à <0,1%.

7. Procédé selon la revendication 1 dans lequel le MADMAP est introduit jusqu'à ce que pratiquement tout le PV de départ ait été copolymérisé en copolymère désiré.

8. Procédé selon la revendication 1 dans lequel le radical initiateur est le t-butylperoxy pivalate.

9. Procédé selon la revendication 6 dans lequel le radical initiateur supplémentaire est le 4,4'-azobis(4-cyano-acide valérique).

10. Procédé selon la revendication 1 dans lequel jusqu'à 10% de MADMAP est inclut dans la précharge.

11. Solution aqueuse claire d'un copolymère homogène de PV et de MADMAP, dans une gamme de composition de 2-99% en poids de PV et 1-80% de MADMAP, en poids, ayant un poids moyen de poids moléculaire de 200000 à 1500000, dont le rapport de composition approche le rapport nominal du monomère de PV:MADMAP et dont la structure a les unités respectives du monomère distribuées le long du squelette du polymère, la solution ayant une teneur en matière sèche de 5-20%, et une viscosité de Brookfield de 5 à 80 Nm$^{-2}$s (5000 à 80000 cps).

12. Solution aqueuse claire, selon la revendication 11 dans laquelle ladite gamme de composition est de 50-95% de PV à 5-50% de MADMAP.

13. Solution aqueuse claire selon la revendication 11 dans laquelle ladite teneur en matière sèche est de 10-15%.

14. Solution aqueuse claire selon la revendication 13 dans laquelle ladite viscosité est de 10 à 70 Nm$^{-2}$s (10000 à 70000 cps).

15. Solution aqueuse claire selon la revendication 11 dans laquelle ladite gamme de composition est de 80:20, en % par poids.

16. Composition utilisée pour la coiffure contenant une solution d'un copolymère homogène de PV et de MADMAP dans la gamme de composition de 20-90% de PV et 1-80% de MADMAP, en poids, selon la revendication 12.

17. Composition utilisée pour la coiffure selon la revendication 16 dans lequel ledit copolymère homogène est préparé selon le procédé de la revendication 1.

**Patentansprüche**

1. Verfahren zur Herstellung klarer, wässriger Lösungen eines homogenen Copolymers von Vinylpyrrolidon (VP) mit N-[3-(Dimethylamino)propyl]methacrylamid (DMAPMA) im Zusammensetzungsbereich von 20 bis 99 Gew.-% VP und 1 bis 80 Gew.-% DMAPMA mit einem gewichtsmittleren Molekulargewicht von 200.000 bis 1.500.000, dessen Zusammensetzungsverhältnis sich dem nominellen Monomerverhältnis zwischen VP und DMAPMA annähert und in dessen Struktur die jeweiligen Monomereinheiten entlang der Hauptkette des Polymers verteilt sind, wobei die Lösung einen Feststoffgehalt von 5 bis 20 % und eine Brookfield-Viskosität von 5 bis 80 Nm$^{-2}$s (5.000 bis 80.000 cp) aufweist, durch Polymerisation der Monomere in Wasser in Gegenwart eines Radikalinitiators, welches Folgendes umfasst:

(a) das Vorlegen von VP und Wasser bei einer Polymerisationstemperatur von 50 bis 80 °C in einem Reaktor, und

(b) das Zuführen von DMAPMA in den Reaktor mit einer Rate, die der Rate des Verschwindens von VP entspricht.

2. Verfahren nach Anspruch 1, worin das DMAPMA in Wasser zugeführt wird.

3. Verfahren nach Anspruch 2, worin der Zusammensetzungsbereich 50 bis 59 % VP zu 5 bis 50 % DMAPMA beträgt.

4. Verfahren nach Anspruch 1, worin der Feststoffgehalt 10 bis 15 % beträgt.

5. Verfahren nach Anspruch 4, worin die Viskosität 20 bis 70 Nm$^{-2}$s (10.000 bis 70.000 cp) beträgt.

6. Verfahren nach Anspruch 1, das auch den Schritt des Nacherhitzens mit zusätzlichem Radikalinitiator umfasst, um den verbleibenden VP-Gehalt auf < 0,1 % zu verringern.

7. Verfahren nach Anspruch 1, worin DMAPMA zugeführt wird, bis im Wesentlichen das gesamte Ausgangs-VP zum gewünschten Copolymer copolymerisiert wurde.

8. Verfahren nach Anspruch 1, worin der Radikalinitiator t-Butylperoxypivalat ist.

9. Verfahren nach Anspruch 6, worin der zusätzliche Radikalinitiator 4,4'-Azobis-(4-cyanovaleriansäure) ist.

10. Verfahren nach Anspruch 1, worin bis zu 10 % des DMAPMA in der Vorlage enthalten sind.

11. Klare, wässrige Lösung eines homogenen Copolymers von VP mit DMAPMA im Zusammensetzungsbereich von 2 bis 99 Gew.-% VP und 1 bis 80 Gew.-% DMAPMA mit einem gewichtsmittleren Molekulargewicht von 200.000 bis 1.500.000, dessen Zusammensetzungsverhältnis sich dem nominellen Monomerverhältnis zwischen VP und DMAPMA annähert und in dessen Struktur die jeweiligen Monomereinheiten entlang der Hauptkette des Polymers verteilt sind, wobei die Lösung einen Feststoffgehalt von 5 bis 20 % und eine Viskosität von 10 bis 80 Nm$^{-2}$s (10.000 bis 80.000 cp) aufweist.

12. Klare, wässrige Lösung nach Anspruch 11, worin der Zusammensetzungsbereich 50 bis 95 % VP zu 5 bis 50 % DMAPMA beträgt.

13. Klare, wässrige Lösung nach Anspruch 11, worin der Feststoffgehalt 10 bis 15 % beträgt.

14. Klare, wässrige Lösung nach Anspruch 13, worin die Viskosität 10 bis 70 Nm$^{-2}$s (10.000 bis 70.000 cp) beträgt.

15. Klare, wässrige Lösung nach Anspruch 11, worin der Zusammensetzungsbereich 80:20 (in Gew.-%) beträgt.

16. Hair-Styling-Zusammensetzung, die eine wässrige Lösung eines homogenen Copolymers von VP mit DMAPMA im Zusammensetzungsbereich von 20 bis 90 Gew.-% VP und 1 bis 80 Gew.-% DMAPMA nach Anspruch 12 enthält.

17. Hair-Styling-Zusammensetzung nach Anspruch 16, worin das homogene Copolymer nach einem Verfahren nach Anspruch 1 hergestellt ist.

FIGURE 1

EP 0 857 054 B1

FIGURE 2